(19) Europäisches Patentamt
European Patent Office
Office européen des brevets

(11) Veröffentlichungsnummer : **0 029 996**
**B1**

(12) # EUROPÄISCHE PATENTSCHRIFT

(45) Veröffentlichungstag der Patentschrift : **16.02.83**

(21) Anmeldenummer : **80107346.1**

(22) Anmeldetag : **25.11.80**

(51) Int. Cl.³ : **C 07 C103/48**, C 07 C103/38, C 07 C102/00, C 07 D307/68, C 07 D233/61, C 07 D407/12, C 07 C103/42, C 07 D317/28, C 07 C149/23, C 07 D249/08, C 07 D261/18

(54) **N-substituierte 2-Methylnaphthylamide, Verfahren zu ihrer Herstellung und diese enthaltende Fungizide.**

(30) Priorität : **04.12.79 DE 2948704**

(43) Veröffentlichungstag der Anmeldung : **10.06.81 Patentblatt 81/23**

(45) Bekanntmachung des Hinweises auf die Patenterteilung : **16.02.83 Patentblatt 83/07**

(84) Benannte Vertragsstaaten : **AT BE CH DE FR GB IT LI LU NL SE**

(56) Entgegenhaltungen :
**EP A 0 018 510**
**DE A 1 768 435**
**DE B 1 003 221**

(73) Patentinhaber : **BASF Aktiengesellschaft**
**Carl-Bosch-Strasse 38**
**D-6700 Ludwigshafen (DE)**

(72) Erfinder : **Rentzea, Costin, Dr.**
**Neuenheimer Landstrasse 72**
**D-6900 Heidelberg (DE)**
Erfinder : **Zeeh, Bernd, Dr.**
**Thorwaldsenstrasse 5**
**D-6700 Ludwigshafen (DE)**
Erfinder : **Pommer, Ernst-Heinrich, Dr.**
**Berliner Platz 7**
**D-6703 Limburgerhof (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 029 996 B1

N-substituierte 2-Methylnaphthylamide, Verfahren zu ihrer Herstellung und diese enthaltende Fungizide

Die vorliegende Erfindung betrifft neue N-substituierte 2-Methylnaphthylamide, Verfahren zu ihrer Herstellung und Fungizide, die diese Verbindungen enthalten.

Es ist bereits bekannt, Zink-ethylen-1,2-bisdithiocarbamidat, das N-Trichlormethylthiophthalimid und das N-Trichlormethylthio-tetrahydro-phthalimid als Fungizide in der Landwirtschaft und im Gartenbau zu verwenden. Die genannten Verbindungen sind gute Mittel zur Bekämpfung von pilzlichen Krankheiten (vgl. R. Wegler, « Chemie der Pflanzenschutz- und Schädlingsbekämpfungsmittel », Band 2, Seiten 65 bis 66 und 109 und Band 4, Seiten 139 und 191, Berlin/Heidelberg/New York, (1970) und (1977)).

Jedoch sind diese Fungizide nicht nach erfolgter Infektion verwendbar und ihre Wirkung bei niedrigen Aufwandkonzentrationen genügt nicht den Anforderungen der Praxis.

Es wurde nun gefunden, daß neue N-substituierte 2-Methylnaphthylamide der allgemeinen Formel I

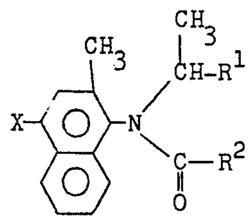

I

worin

$R^1$ eine

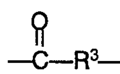Gruppe

bedeutet, wobei $R^3$ Wasserstoff oder $C_1$-$C_4$-Alkoxy bedeutet und weiterhin $R^1$ eine

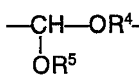Gruppe

bedeutet, wobei $R^4$ und $R^5$ unabhängig voneinander einen $C_1$-$C_4$-Alkylrest oder $R^4$ und $R^5$ zusammen eine Alkylengruppe bedeuten, die zusammen mit dem Rest, dessen Substituenten sie sind, einen gegebenenfalls durch $C_1$-$C_4$-Alkyl oder Aryl substituierten 5- oder 6-gliedrigen zwei Sauerstoffatome enthaltenden heterocyclischen Ring bildet und $R^2$ einen gegebenenfalls durch Fluor, Chlor, Brom, Jod, $C_1$-$C_4$-Alkoxy, $C_1$-$C_4$-Alkylthio, 1-Imidazolyl, 1-Pyrazolyl, 1,2,4-Triazol-(1)- oder Oxo (= O)-substituierten $C_1$-$C_4$-Alkyl-, ferner einen $C_2$-$C_5$-Alkenyl-, $C_2$-$C_5$-Alkinyl-, $C_3$-$C_7$-Cycloalkyl-, $C_4$-$C_7$-Cycloalkenyl-, $C_1$-$C_5$-Alkoxyrest, einen Phenyl- oder gegebenfalls durch Methyl substituierten Heteroarylrest bedeutet und X Wasserstoff, Methyl, Chlor oder Brom bedeutet, mit der Maßgabe, daß nicht gleichzeitig $R^3$ $C_1$-$C_4$-Alkoxy, X Wasserstoff und $R^2$ einen 2-Furylrest, einen $C_1$-$C_4$-Alkoxy-$C_1$-$C_2$-alkylrest, einen $C_1$-$C_4$-Alkylthio-$C_1$-$C_2$-alkylrest, einen -$CH_2$-1,2-pyrazolylrest oder einen -$CH_2$-1,2,4-Triazolylrest bedeuten, starke fungizide Eigenschaften aufweisen.

In der Formel I steht $R^2$ bevorzugt für einen unverzweigten oder verzweigten $C_1$-$C_5$-Alkoxyrest, z. B. Methoxy, Ethoxy, n-Propoxy, iso-Propoxy, n-Butoxy, iso-Butoxy, sek.-Butoxy, tert.-Butoxy, n-Pentyloxy oder iso-Pentyloxy, oder für einen gegebenenfalls durch Fluor, Chlor, Brom, Jod, Methoxy, Ethoxy, Butoxy, Methylthio-, Ethylthio, 1-Imidazolyl, 1-Pyrazolyl oder 1,2,4-Triazolyl-(1) substituierten, verzweigten oder unverzweigten $C_1$-$C_4$-Alkylrest, z. B. Methyl, Ethyl, n-Propyl, iso-Propyl, n-Butyl, iso-Butyl, sek.-Butyl, oder tert.-Butyl.

$R^2$ steht ferner beispielsweise für Vinyl, 1-Propenyl, 2-Propenyl, Allyl, Ethinyl, oder beispielsweise für einen cyclischen Alkyl- oder Alkenylrest mit 3 bis 7 Ringkohlenstoffatomen, beispielsweise Cyclopropyl, Cyclopentyl, Cyclohexyl oder Cyclohexen-2-yl, ferner beispielsweise für einen Phenylrest oder für einen gegebenenfalls durch Methyl substituierten Heteroarylrest mit 5 bis 6 Ringatomen und 1 bis 3, insbesondere

1 bis 2 Heteroatomen im Ring, die gleich oder verschieden sein können und vorzugsweise Sauerstoff, Stickstoff oder Schwefel sind, wie beispielsweise einen Furan-, Thiophen-, Isoxazol- oder Pyridinrest.

$R^3$ steht insbesondere für Wasserstoff und für einen verzweigten oder unverzweigten $C_1$-$C_4$-Alkoxyrest, z. B. Methoxy, Ethoxy, n-Propoxy, iso-Propoxy, n-Butoxy, iso-Butoxy, sek.-Butoxy und tert.-Butoxy.

$R^4$ und $R^5$ stehen beispielsweise für einen gegebenenfalls substituierten unverzweigten oder verzweigten Alkylrest mit bis zu 4 Kohlenstoffatomen, beispielsweise Methyl, Ethyl, n-Propyl, iso-Propyl, n-Butyl oder iso-Butyl. $R^4$ und $R^5$ stehen ferner beispielsweise zusammen für eine Alkylengruppe mit 2 oder 3 Kohlenstoffatomen, die gegebenenfalls durch Alkyl mit bis zu 4 Kohlenstoffatomen, beispielsweise Methyl oder Ethyl, substituiert sein kann. X steht vorzugsweise für Wasserstoff, Methyl, Chlor oder Brom.

Die neuen N-substituierten 2-Methylnaphthylamide der Formel I besitzen ein chirales Kohlenstoffatom mit H, $CH_3$, $R^1$ und -N- als Liganden und je nach der Beschaffenheit von $R^2$, $R^3$, $R^4$ und $R^5$ weitere Chiralitätszentren. Nach üblichen Methoden können die optisch reinen Enantiomeren bzw. die Diastereomeren erhalten werden. Die vorliegende Erfindung erfaßt auch diese Verbindungen in reiner Form oder als Mischungen. Als Fungizide sind sowohl die reinen Enantiomeren bzw. die einheitlichen Diastereomeren als auch die bei der Synthese üblicherweise anfallenden Mischungen wirksam.

Weiterhin wurde gefunden, daß man die neuen Verbindungen der allgemeinen Formel (I) erhält, wenn man ein 2-Methylnaphthylamin der Formel II

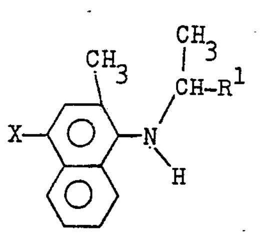

II

in welcher

$R^1$ und X die oben genannten Bedeutungen haben,

a) mit einem Säurehalogenid der Formel III

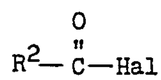

III

oder

b) mit einem Säureanhydrid der Formel IV

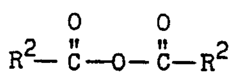

IV

in welcher

$R^2$ die oben genannte Bedeutung hat und Hal Chlor oder Brom bedeutet, gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers, bei Temperaturen zwischen − 10 und 100 °C umsetzt. Diese Umsetzung wird bevorzugt. Als bevorzugte, gegenüber den Reaktionsteilnehmern inerte Lösungs- oder Verdünnungsmittel werden z. B. aliphatische oder aromatische Kohlenwasserstoffe wie Pentan, Cyclohexan, Petrolether, Benzol, Toluol oder Xylole ; Halogenkohlenwasserstoffe, beispielsweise Methylenchlorid, Chloroform, 1,2-Dichlorethan, Chlorbenzol ; Ketone wie Aceton und Methylethylketon ; Ether wie Diethylether, Dimethoxyethan, Tetrahydrofuran oder Dioxan ; Ester wie Essigsäureethylester ; Nitrile wie Acetonitril ; Sulfoxide wie Dimethylsulfoxid oder entsprechende Gemische verwendet.

Geeignete anorganische oder organische Basen, die gegebenenfalls auch als säurebindende Mittel bei der Reaktion verwendet werden können, sind beispielsweise Alkali- und Erdalkalicarbonate wie Natrium- oder Kaliumbicarbonat, Natrium- oder Kaliumcarbonat, Calciumcarbonat ; Borate wie Natriumborat ; Phosphate wie Natrium- oder Kaliumdi- oder -triphosphat oder Amine wie Triethylamin, N,N-Dimethylanilin, N,N-Dimethylcyclohexylamin, N-Methylpiperidin oder Pyridin. Es können aber auch andere übliche Basen verwendet werden.

Als Reaktionsbeschleuniger kommen vorzugsweise Metallhalogenide wie Natriumbromid oder Kaliumjodid, Azole wie Imidazol oder 1,2,4-Triazol oder Pyridine wie 4-Dimethylaminopyridin in Frage.

Die erfindungsgemäßen Umsetzungen erfolgen beispielsweise bei Temperaturen zwischen − 10 und + 100 °C, vorzugsweise zwischen 0 und + 40 °C, drucklos oder unter Druck, kontinuierlich oder diskontinuierlich.

Es wurde ferner gefunden, daß Verbindungen der Formel I erhalten werden, indem man ein 2-Methylnaphthylamid der Formel Ia

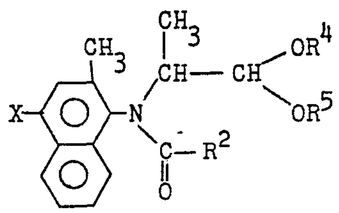

Ia

in welcher

$R^2$ und X die oben genannten Bedeutungen haben und $R^4$ und $R^5$ unabhängig voneinander einen gegebenenfalls substituierten $C_1$-$C_4$-Alkylrest bedeuten, durch Abspaltung von $R^4$ und $R^5$ zu dem entsprechenden Aldehyd oder durch Umsetzung mit einem dem Diol HO—$R^4$—$R^5$—OH, wobei $R^4$ und $R^5$ zusammen eine Alkylengruppe bedeuten, zu dem entsprechenden cyclischen Acetal, gegebenenfalls in Gegenwart eines Verdünnungsmittels und gegebenenfalls unter Zusatz eines sauren Katalysators, umsetzt.

Zu dem oben genannten Diol gehören beispielsweise Ethylenglykol, 1,2-Propylenglykol, 1,3-Propandiol, 2,3-Butandiol und Neopentylglykol. Die Abspaltung von $R^4$ und $R^5$ wird beispielsweise vorgenommen, indem man katalytische Mengen von starken protischen oder Lewis-Säuren wie Chlorwasserstoff (bzw. Salzsäure), Bromwasserstoff, p-Toluolsulfonsäure, Trifluoressigsäure, Trifluormethylsulfonsäure, Zinkchlorid, Zinkbromid oder Bortrifluorid-etherat bei Temperaturen von 0 °C und bis zum Siedepunkt der abgespalteten Alkohole $R_4$-OH und $R_5$-OH einwirken läßt. Die Umsetzung mit einem 1,2- oder 1,3-Diol wird beispielsweise vorgenommen, indem man die Alkohole $R_4$—OH und $R_5$—OH aus dem Reaktionsgemisch durch Destillation im Vakuum oder bei atmosphärischem Druck oder mit Hilfe eines inerten Gases, wie Stickstoff oder Argon, entfernt. Als Lösungs- oder Verdünnungsmittel eignen sich die oben genannten Flüssigkeiten oder Wasser, oder die 1,2- oder 1,3-Diole selbst bei Anwendung in stöchiometrischer Menge oder im Überschuß.

Als saure Katalysatoren eignen sich Lewis- oder Protonensäuren, beispielsweise $BF_3$, $AlCl_3$ oder $ZnCl_2$ oder Mineralsäuren oder Sulfonsäuren.

Ferner erhält man Verbindungen der allgemeinen Formel I, indem man ein 2-Methylnaphthylamid der Formel Ib

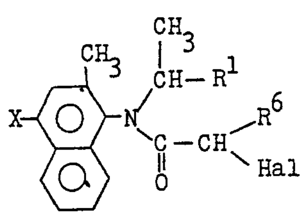

Ib

in welcher

$R^1$ und X die oben angegebenen Bedeutungen haben, Hal ein Halogenatom bedeutet und $R^6$ Wasserstoff, einen $C_1$-$C_3$-Alkyl-, $C_2$-$C_4$-Alkenyl-, $C_3$-$C_7$-Cycloalkyl- oder $C_1$-$C_5$-Alkoxyrest bedeutet mit einer nucleophilen Verbindung der Formel V

NuH V

worin Nu einen $C_1$-$C_5$-Alkoxy- oder $C_1$-$C_4$-Alkylthio- oder 1-Imidazolyl-, 1-Pyrazolyl- oder 1,2,4-Triazolyl-(1)-Rest bedeutet, gegebenenfalls in Gegenwart eines Lösungs- oder Verdünnungsmittels, gegebenenfalls in Gegenwart einer anorganischen oder organischen Base und gegebenenfalls unter Zusatz eines Reaktionsbeschleunigers bei Temperaturen zwischen − 10 und + 100 °C umsetzt.

Weitere Verbindungen der allgemeinen Formel I erhält man, wenn man je nach der funktionellen Beschaffenheit von $R^1$ und $R^2$ übliche chemische Folgereaktionen vornimmt.

Die als Ausgangsstoffe zur Herstellung der Verbindungen der Formel I verwendeten 2-Methylnaphthylamine der Formel II erhält man, wenn man ein 2-Methylnaphthylamin der Formel VI

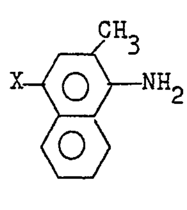

VI

worin X die oben angegebene Bedeutung hat,

a) mit einem 2-Halogen-propionsäureester der Formel VII

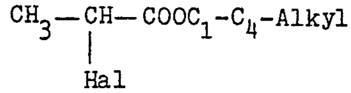

VII

in der Hal Chlor oder Brom bedeutet, oder mit einem 2-Halogen-propanaldialkylacetal der Formel III

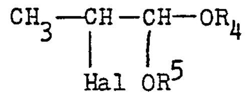

VIII

worin Hal Chlor oder Brom bedeutet und $R^4$ und $R^5$ die oben angegebenen Bedeutungen haben, gegebenenfalls in Gegenwart eines Lösungs- oder Verdünnungsmittels, gegebenenfalls in Gegenwart einer anorganischen oder organischen Base und gegebenenfalls unter Zusatz eines Reaktionsbeschleunigers bei Temperaturen zwischen − 10 und + 130 °C umsetzt.

Zu den bevorzugten Lösungs- und Verdünnungsmitteln, zu den anorganischen oder organischen Base und zu den Reaktionsbeschleunigern gehören die oben genannten Verbindungen.

Es wurde weiterhin gefunden, daß man die Verbindungen der allgemeinen Formel II erhält, wenn man

a) ein 2-Methylnaphthylamin der Formel IIb mit einem Brenztraubensäureester der Formel IX

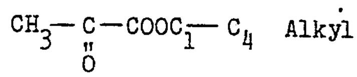

IX

oder einem Methylglyoxalacetal der Formel XI

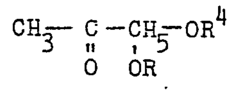

XI

worin $R^4$ oder $R^5$ die oben erläuterten Bedeutungen haben, umsetzt und

b) die als Umsetzungsprodukt erhaltene Schiffsche Base hydriert, z. B. mit komplexen Metallhydriden oder auf katalytischem Wege mit Wasserstoff.

Die Herstellung der Schiff'schen Base wird beispielsweise so durchgeführt, daß man 1 Mol des 2-Methylnaphthalamins der Formel VI mit 0,9 bis 1,5 Mol des Brenztraubensäureesters der Formel IX oder des Methylglyoxalacetals der Formel XI in einem Lösungsmittel gegebenenfalls unter Zusatz eines sauren Katalysators umsetzt und bei einer Temperatur von 40 bis 200 °C, vorzugsweise 50 bis 120 °C Wasser durch Destillation abtrennt. Zweckmäßig verwendet man unter den Reaktionsbedingungen inerte und gleichzeitig mit Wasser Azeotrope bildende Lösungsmittel für die Umsetzung. Als Lösungsmittel kommen z. B. in Frage aromatische Kohlenwasserstoffe, beispielsweise Benzol, Toluol, Ethylbenzol, o-, m-, p-Xylol, Isopropylbenzol, Methylnaphthalin ; aliphatische oder cycloaliphatische Kohlenwasserstoffe, beispielsweise Heptan, Nonan, Pinan, Benzinfraktionen innerhalb eines Siedepunktintervalls von 70 bis 190 °C, Cyclohexan, Methylcyclohexan, Dekalin, Ligroin, 2,2,4-Trimethylpentan, 2,2,3-Trimethylpentan, 2, 3,3-Trimethylpentan, Octan und entsprechende Gemische.

Für die Hydrierung eignen sich sowohl die Reduktion mit komplexen Hydriden wie $NaBH_4$ als auch katalytische Hydrierung mit Wasserstoff.

Die Reduktion mit Natriumborhydrid wird im allgemeinen so durchgeführt, daß man in einem Lösungsmittel die Schiff'schen Basen mit 0,2 Mol bis 1 Mol Natriumborhydrid pro Mol Schiff'sche Base bei einer Temperatur zwischen − 20 und + 40 °C umsetzt.

Bei der katalytischen Hydrierung werden dem Reaktionsgemisch am Anfang und im Verlauf der Umsetzung solche Mengen an Wasserstoff zugeführt, daß sich bei der Umsetzungstemperatur stets ein entsprechender Reaktionsdruck, zweckmäßig zwischen 150 und 300 bar, einstellt. Die Umsetzung wird im allgemeinen bei einer Temperatur von 20 bis 200 °C, vorzugsweise von 25 bis 160 °C, diskontinuierlich oder kontinuierlich durchgeführt. Zur entsprechenden Druckeinstellung können auch inerte Gase wie Stickstoff verwendet werden.

Für beide Verfahrensvarianten der Hydrierung eignen sich als Lösungs- oder Verdünnungsmittel besonders gut Alkanole und Cycloalkanole, z. B. n-Propanol, Isopropanol, n-Butanol, Isobutanol, Glykol, Ethylenglykolmonoethylether, Glycerin, Amylalkohol, Cyclohexanol, 2-Methyl-4-pentanol, 2-Ethylhexanol und insbesondere Methanol, Ethanol ; cyclische Ether wie Tetrahydrofuran und Dioxan.

Zur katalytischen Hydrierung wird der Katalysator in der Regel in einer Menge von 5 bis 30 Gewichtsprozent, bezogen auf die Schiff'sche Base, verwendet. Er kann im Gemisch mit einem für die Umsetzung geeigneten Trägermaterial, z. B. Siliciumdioxid, zur Anwendung gelangen, wobei zweckmäßig die Menge des Katalysators 10 bis 40 Gewichtsprozent des Gemisches von Katalysator und Träger beträgt.

Zweckmäßig verwendet man Kupferchromitkatalysatoren, z. B. Kupferchromoxidkatalysatoren wie die von H. Adkins (vgl. Houben-Weyl, Methoden der organischem Chemie, Band 4/2, Seiten 180 bis 183 und J. Applied Chem. *5*, 289-295 (1955)) verwendeten Kupferchromite. Sie enthalten beispielsweise Kupfer-Chrom-Spinell ($CuCr_2O_4$) oder Gemische im Verhältnis 5 CuO : 4 $Cr_2O_3$ bzw. gehen von solchen Verbindungen aus und können noch andere Oxide, hauptsächlich die der Erdalkalimetalle wie Magnesium, Calcium oder Barium enthalten.

Folgende Verfahrensbeschreibungen erläutern die Herstellung von 2-Methylnaphthylaminen der Formel II :

a) Direkte Alkylierung von 1-Amino-2-methylnaphthalin

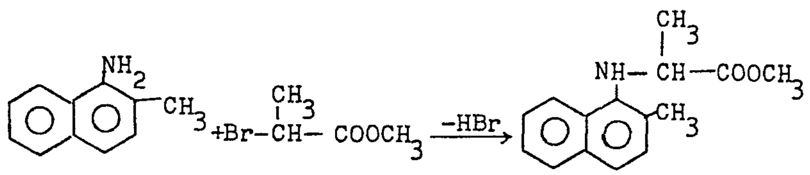

(1)

144,2 g 1-Amino-2-methylnaphthalin (0,92 Mol) werden mit 90,2 g (1,08 Mol) Natriumhydrogencarbonat und 460 g (2,76 Mol) 2-Brompropionsäuremethylester bei 120 bis 125 °C 18 Stunden gerührt. Nach dem Abkühlen wird der Niederschlag abgesaugt und das Filtrat im Vakuum eingeengt. Der Rückstand wird im Vakuum destilliert. Man erhält 188 g (83,7 % d. Th.) N-(2-Methyl-1-naphthyl)-alanin-methylester als farbloses Öl. Sdp. : 138-140 °C/0,2 mbar.

b) Schiff'sche Base des 1-Amino-2-methylnaphthalins

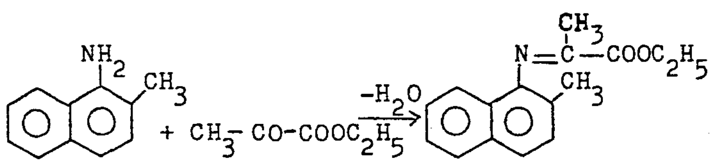

(2)

314 g 1-Amino-2-methylnaphthalin (2 Mol), 232 g Brenztraubensäure-ethylester (2 Mol) und 0,4 g p-Toluolsulfonsäure werden in 1 000 ml Cyclohexan für 4 Stunden am Rückfluß erhitzt bis 36 g Wasser azeotrop abdestilliert und aus dem Destillat abgetrennt sind. Dann wird das Cyclohexan im Vakuum abdestilliert und der Rückstand direkt weiter umgesetzt.

Ausbeute : 494,7 g (97 % d. Th.) Schiff'sche Base.

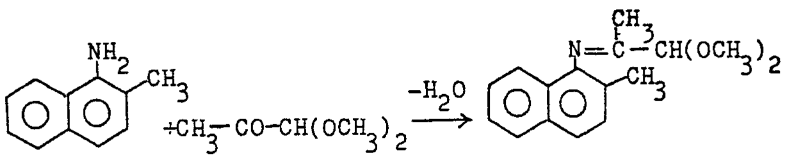

(3)

314 g 1-Amino-2-methylnaphthalin, 236 g Methylglyoxaldimethylacetal und 0,4 g p-Toluolsulfonsäure werden analog Verfahren b, Gleichung (2) umgesetzt.

Ausbeute : 493,4 g (96 % d. Th.) Schiff'sche Base.

c) Hydrierung der Schiff'schen Base

Katalytische Hydrierung

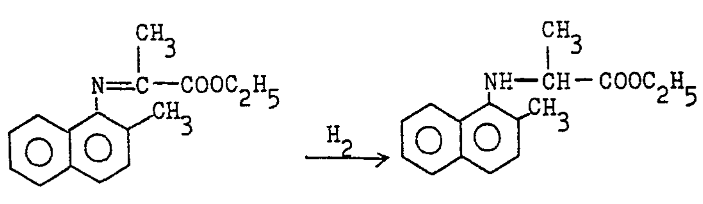

(4)

Ein Hydrierautoklav mit einem Volumen von 1 l wird mit 200 g der Schiff'schen Base aus 1-Amino-2-methylnaphthalin und Brenztraubensäuremethylester, die in 500 Teilen Tetrahydrofuran gelöst sind, und 15 Teilen Adkins-Katalysator (Kupferchromit in pulverisierter Form) gefüllt. Anschließend wird der Autoklav auf 150 °C erhitzt und Wasserstoff bis zum Erreichen eines Druckes von 200 bar aufgepreßt. Sobald die Wasserstoffaufnahme beendet und ein konstanter Druck erreicht ist (nach ca. 9 Stunden), wird abgekühlt, der Katalysator abgesaugt und das Filtrat im Vakuum destilliert.

Man erhält 142 g (71 % d. Th.) N-(2-Methyl-1-naphthyl)-alanin-ethylester als farbloses Öl vom Sdp. 144-145 °C/0,15 mbar.

Reduktion mit $NaBH_4$

490 g der Schiff'schen Base aus 1-Amino-2-methylnaphthalin und Methylglyoxaldimethylacetal werden in 1 500 ml Methanol gelöst und bei 0 °C portionsweise mit 60 g Natriumborhydrid versetzt. Nach dem Rühren über Nacht wird das Methanol abdestilliert, der Rückstand in 1 000 ml Methylenchlorid gelöst und 30 Minuten mit 500 ml 10 %iger Kalilauge gerührt und die organische Phase abgetrennt. Nach dreimaligem Waschen mit je 200 ml Wasser und Trocknen über Natriumsulfat wird das Methylenchlorid abdestilliert und der Rückstand im Vakuum destilliert.

Man erhält 367 g (74 % d. Th.) 2-(N-2'-Methyl-1'-naphthyl)-aminopropanaldimethylacetal vom Sdp. 140-145 °C/0,3 mbar.

2-N-Cyclopropylcarbonyl-N-(2-methyl-1-naphthyl)-alanin-methylester,
2-N-Cyclopropylcarbonyl-N-(2-methyl-1-naphthyl)-alanin-ethylester,
2-N-Cyclopentylcarbonyl-N-(2-methyl-1-naphthyl)-alanin-methylester,
2-N-Cyclohexylcarbonyl-N-(2-methyl-1-naphthyl)-alanin-methylester,
2-N-Methoxycarbonyl-N-(2-methyl-1-naphthyl)-alanin-methylester,
2-N-Ethoxycarbonyl-N-(2-methyl-1-naphthyl)-alanin-methylester,
2-N-Ethoxycarbonyl-N-(2-methyl-1-naphthyl)-alanin-ethylester,
2-N-Propoxycarbonyl-N-(2-methyl-1-naphthyl)-alanin-methylester,
2-N-Allyloxycarbonyl-N-(2-methoxy-1-naphthyl)-alanin-methylester,
2-N-Butoxycarbonyl-N-(2-methoxy-1-naphthyl)-alanin-methylester,
2-N-Methoxalyl-N-(2-methoxy-1-naphthyl)-alanin-methylester,
2-N-Ethoxalyl-N-(2-methoxy-1-naphthyl)-alanin-methylester,
2-N-Ethoxalyl-N-(2-methyl-1-naphthyl)-alanin-ethylester,
2-N-Acetoacetyl-N-(2-methyl-1-naphthyl-alanin-methylester,
2-N-(4-Oxo-pentanoyl)-N-(2'-methyl-1'-naphthyl)-alanin-methylester,
2-N-(3-Hydroxybutyryl)-N-(2'-methyl-1'-naphthyl)-alanin-methylester,
2-N-Benzoyl-N-(2-methyl-1-naphthyl)-alanin-methylester,
2-N-(2-Chlor-benzoyl)-N-(2'-methyl-1'-naphthyl)-alanin-methylester,
2-N-(2-Brom-benzoyl)-N-(2'-methyl-1'-naphthyl)-alanin-methylester,
2-N-(2-Jod-benzoyl)-N-(2'-methyl-1'-naphthyl)-alanin-methylester,
2-N-(2-Methylbenzoyl)-N-(2'-methyl-1'-naphthyl)-alanin-methylester,
2-N-(2-Trifluormethyl-benzoyl)-N-(2'-methyl-1'-naphthyl)-alanin-methylester,
2-N-(2-Methoxy-benzoyl)-N-(2'-methyl-1'-naphthyl)-alanin-methylester,
2-N-(2-Thiophen-carbonyl)-N-(2'-methyl-1'-naphthyl)-alanin-methylester,
2-N-(2-Methylfuran-3-carbonyl)-N-(2'-methyl-1'-naphthyl)-alanin-methylester,
2-N-(2,5-Dimethylfuran-3-carbonyl)-N-(2'-methyl-1'-methyl-1'-naphthyl)-alanin-methylester,
2-N-(2,4,5-Trimethylfuran-3-carbonyl)-N-(2'-methyl-1'-naphthyl)-alanin-methylester,
2-N-(3-Isoxazolyl-carbonyl)-N-(2'-methyl-1'-naphthyl)-alanin-methylester,
2-N-(3-Isoxazolyl-carbonyl)-N-(2'-methyl-1'-naphthyl)-alanin-ethylester,
2-N-(3-Methylisoxazolyl-5-carbonyl)-N-(2'-methyl-1'-naphthyl)-alanin-methylester,
2-N-Nicotinoyl-N-(2-methyl-1-naphthyl)-alanin-methylester,
2-N-(2-Chlor-nicotinoyl)-N-(2'-methyl-1'-naphthyl)-alanin-methylester,
2-N-Acryloyl-N-(2-methyl-1-naphthyl)-alanin-methylester,
2-N-(2-Methylacryloyl)-N-(2-methyl-1-naphthyl)-alanin-methylester,
2-N-Phenoxycarbonyl-N-(2-methyl-1-naphthyl)-alanin-methylester,
2-N-(4-Chlorphenoxy-carbonyl)-N-(2'-methyl-1-naphthyl)-alanin-methylester,
2-[N-(2-Methyl-1-naphthyl)-N-acetyl]-aminopropanoldimethylacetal
2-[N-(2-Methyl-1-naphthyl)-N-propionyl]-aminopropanaldimethylacetal,
2-[N-(2-Methyl-1-naphthyl)-N-butyryl]-aminopropanaldimethylacetal,
2-[N-(2-Methyl-1-naphthyl)-N-chloracetyl]-aminopropanaldimethylacetal,
Chloressigsäure-N-[1-(1,3-dioxolan-2-yl)-ethyl]-N-2'-methyl-1'-naphthylamid,
Chloressigsäure-N-[1-(4'-methyl-1',3'-dioxolan-2'-yl)-ethyl]-N-2« -methyl-1 »-naphthylamid,
2-[N-(2-Methyl-1-naphthyl)-N-bromacetyl]-aminopropanaldimethylacetal,
2-[N-(2-Methyl-1-naphthyl)-N-jodacetyl]-aminopropanaldimethylacetal,
2-[N-(2-Methyl-1-naphthyl)-N-methoxyacetyl]-aminopropanaldimethylacetal,
Methoxyessigsäure-N-[1,3-dioxolan-2-yl)-ethyl]-N-2'-methyl-1'-naphthylamid,
Methoxyessigsäure-N-[1-(4'-methyl-1',3'-dioxolan-2'-yl)-ethyl]-N-2"-methyl-1"-naphthylamid,
Methoxyessigsäure-N-[1-(4',5'-dimethyl-1',3'-dioxolan-2'-yl)-ethyl]-N-2"-methyl-1"-naphthylamid,
2-[N-(2-Methyl-1-naphthyl)-N-ethoxyacetyl]-aminopropanaldimethylacetal,
2-[N-(2-Methyl-1-naphthyl)-N-methylthioacetyl]-aminopropanaldimethylacetal,
2-[N-(2-Methyl-1-naphthyl)-N-(2'-chlorpropionyl)]-aminopropanaldimethylacetal,
2-[N-(2-Methyl-1-naphthyl)-N-(1'-imidazolylacetyl)]-aminopropanoldimethylacetal,
2-[N-(2-Methyl-1-naphthyl)-N-(1'-pyrazolylacetyl)]-aminopropanaldimethylacetal,
2-[N-(2-Methyl-1-naphthyl)-N-(1',2',4'-triazolyl-(1')-acetyl)]-aminopropanaldimethylacetal,
2-[N-(2-Methyl-1-naphthyl)-N-cyclopropylcarbonyl]-aminopropanaldimethylacetal,
2-[N-(2-Methyl-1-naphthyl)-N-Cyclohexylcarbonyl]-aminopropanaldimethylacetal,

2-[N-(2-Methyl-1-naphthyl)-N-methoxycarbonyl]-aminopropanaldimethylacetal,
2-[N-(2-Methyl-1-naphthyl)-N-ethoxycarbonyl]-aminopropanaldimethylacetal,
2-[N-(2-Methyl-1-naphthyl)-N-butoxycarbonyl]-aminopropanaldimethylacetal,
2-[N-(2-Methyl-1-naphthyl)-N-allyloxycarbonyl]-aminopropanaldimethylacetal,
2-[N-(2-Methyl-1-naphthyl)-N-methoxalyl]-aminopropanaldimethylacetal,
2-[N-(2-Methyl-1-naphthyl)-N-ethoxalyl]-aminopropanaldimethylacetal,
2-[N-(2-Methyl-1-naphthyl)-N-acetoacetyl]-aminopropanaldimethylacetal,
Acetessigsäure-N-[1-(1,3-dioxolan-2-yl)-ethyl]-N-2'-methyl-1'-naphthylamid,
2-[N-(2-Methyl-1-naphthyl)-N-(3'-hydroxybutyryl)]-aminopropanaldimethylacetal,
2-[N-(2-Methyl-1-naphthyl)-N-benzoyl]-aminopropanaldimethylacetal,
2-[N-(2-Methyl-1-naphthyl)-N-(2'-chlorbenzoyl)]-aminopropanaldimethylacetal,
2-[N-(2-Methyl-1-naphthyl)-N-(2'-brombenzoyl)]-aminopropanaldimethylacetal,
2-[N-(2-Methyl-1-naphthyl)-N-(2'-trifluormethyl-benzoyl)]-aminopropanaldimethylacetal,
2-[N-(2-Methyl-1-naphthyl)-N-(2'-methoxy-benzoyl)]-aminopropanaldimethylacetal,
2-[N-(2-Methyl-1-naphthyl)-N-(2-furancarbonyl)]-aminopropanaldimethylacetal,
2-Furancarbonsäure-N-[1,3-dioxolan-2-yl)-ethyl]-N-2'-methyl-1'-naphthylamid,
2-[N-(2-Methyl-1-naphthyl)-N-(2'-methylfuran-3'-carbonyl)]-aminopropanaldimethylacetal,
2-[N-(2-Methyl-1-naphthyl)-N-(2',4',5'-trimethyl-furan-3'-carbonyl)]-aminopropanaldimethylacetal,
2-[N-(2-Methyl-1-naphthyl)-N-(3'-isoxazolylcarbonyl)]-aminopropanaldimethylacetal,
2-[N-(2-Methyl-1-naphthyl)-N-(3'-methylisoxazolyl-5'-carbonyl)]-aminopropanaldimethylacetal,
2-[N-(2-Methyl-1-naphthyl)-N-nicotinoyl]-aminopropanaldimethylacetal,
2-[N-(2-Methyl-1-naphthyl)-N-(2'-chlornicotinoyl)]-aminopropanaldimethylacetal,
2-[N-(2-Methyl-1-naphthyl)-N-acryloyl]-aminopropanaldimethylacetal,
2-[N-(2-Methyl-1-naphthyl)-N-(4'-chlorphenoxycarbonyl)]-aminopropanaldimethylacetal,
N-(2-Furancarbonyl)-N-(2'-methyl-4'-brom-1'-naphthyl)-alanin-methylester,
N-Methoxyacetyl-N-(2-methyl-4-brom-1-naphthyl)-alanin-methylester,
2-[N-(2-Methyl-1-naphthyl)-N-bromacetyl]-aminopropionaldehyd,
2-[N-(2-Methyl-1-naphthyl)-N-methoxyacetyl]-aminopropionaldehyd.

Die Herstellung der neuen Verbindungen wird durch folgende Beispiele erläutert :

## Beispiel 1

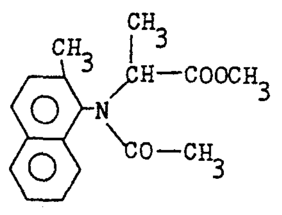

Ein Gemisch von 30,7 g (0,12 Mol) N-(2-Methyl-1-naphthyl)-alanin-methylester, 9,5 g Pyridin (0, 12 Mol), 5 g 4-Dimethylaminopyridin und 50 ml Acetanhydrid wird bei 80 °C 8 Stunden gerührt, dann abgekühlt und im Vakuum eingeengt. Der Rückstand wird mit 250 ml Ethylether und mit 250 ml Wasser versetzt. Die organische Phase wird abgetrennt, mit 100 ml 1-N-Salzsäure, dann mit 100 ml Wasser gewaschen, mit Kohle entfärbt und eingeengt. Der Rückstand wird mit 30 ml eines 5 : 1 Petrolether : Ether-Gemisches bei 0 °C gerührt, 4 Stunden bei 0 °C auskristallisieren lassen und abgesaugt. Man erhält 23,6 g (69 % d. Th.) N-Acetyl-N-(2-Methyl-1-naphthyl)-alanin-methylester als weiße Kristalle vom Schmelzpunkt 119-121 °C.

## Beispiel 2

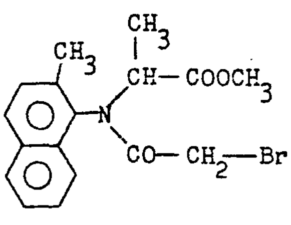

Einer gut gerührten Suspension von 14 g Natriumhydrogencarbonat (0,17 Mol) und 20 g Natriumsulfat in 150 ml trockenem Toluol werden 19,4 g (0,09 Mol) N-(2-Methyl-1-naphthyl)-alanin-methylester zugegeben und anschließend wird eine Lösung von 24,2 g (0,12 Mol) Bromacetylbromid ind 50 ml Toluol in 1 Stunde bei + 15 °C zugetropft. Nach fünfstündigem Rühren bei 25 °C wird der Niederschlag abgesaugt und mit 50 ml Toluol gewaschen. Das Filtrat wird zweimal mit je 100 ml 1N-Natronlauge,

einmal mit 50 ml 2N-Salzsäure und schließlich mit 100 ml Wasser gewaschen, getrocknet, mit Kohle entfärbt und im Vakuum eingedampft. Der verbleibende ölige Rückstand wird 3 Stunden bei 50 °C und 0,1 mbar getrocknet. Man erhält 22,5 g (77,3 % d. Th.) N-Bromacetyl-N-(2-Methyl-1-naphthyl)-alanin-methylester als hellgelbes Öl.

IR-Spektrum (Film) : 3 035, 2 970, 2 935, 1 730, 1 652, 1 440, 1 415, 1 360, 1 200, 1 132, 1 095, 812, 782, 744 $cm^{-1}$.

Beispiel 3

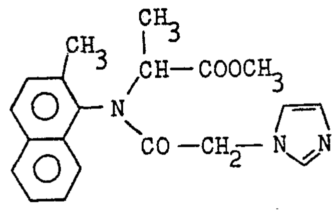

13 g (0,036 Mol) N-Bromacetyl-N-(2-methyl-1-naphthyl)-alanin-methylester (Beispiel 2) werden in 60 ml trockenem Dimethylformamid gelöst und mit 6,8 g (0,1 Mol) Imidazol versetzt. Das Gemisch wird 10 Stunden bei 60 °C gerührt und im Vakuum eingeengt. Der Rückstand wird mit 150 ml Methylenchlorid und 50 ml Wasser ausgeschüttelt, die organische Schicht abgetrennt und zweimal mit je 50 ml Wasser gewaschen. Die organische Lösung wird getrocknet, mit Kohle entfärbt und eingeengt. Nach dem Reinigen des Rohproduktes durch Filtration seiner Lösung in Essigester über Kieselgel erhält man durch Abdampfen des Essigesters als Rückstand 9,6 g Harz.

Ber. : C 68,4 H 6,0 N 12,0 %
Gef. : C 68,0 H 6,4 N 11,9 %.

Beispiel 4

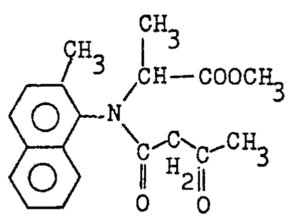

24,3 g (0,1 Mol) N-(2-Methyl-1-naphthyl)-alanin-methylester und 0,5 ml Triethylamin in 50 ml trockenem Toluol werden bei 80 bis 90 °C unter Rühren mit 9 g (0,1 Mol) Diketen tropfenweise versetzt. Nach dreistündigem Rühren bei 95 °C wird das Gemisch abgekühlt, einmal mit 50 ml 2N-Salzsäure und einmal mit Wasser gewaschen, getrocknet und eingeengt. Nach dem Reinigen des Rohproduktes durch Filtration seiner Lösung in Essigester über Kieselgel erhält man durch Abdampfen des Essigesters als Rückstand 29,7 g analysenreines Öl (Ausbeute 90 % d. Th.).

IR-Spektrum (Film) : 3 042, 2 980, 2 940, 1 740, 1 720, 1 650, 1 650, 1 455, 1 370, 1 310, 1 195, 920, 815, 786, 746 $cm^{-1}$.

Beispiel 5

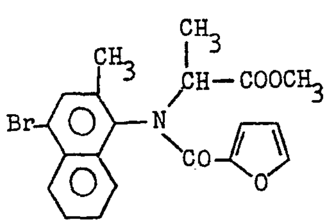

22,2 g (0,066 Mol) N-(2-Furancarbonyl)-N-(2-Methyl-1-naphthyl)-alanin-methylester (Beispiel 21) und 6 g (0,07 Mol) wasserfreies Natriumacetat in 150 ml Essigsäure werden mit 11 g (0,067 Mol) Brom versetzt und 24 Stunden bei 35 °C gerührt. Das Gemisch wird anschließend in 700 ml Eiswasser eingerührt und das Produkt dreimal mit je 100 ml Ether extrahiert. Die vereinigten etherischen Extrakte werden einmal mit 100 ml 1N-Natronlauge und einmal mit Wasser gewaschen, getrocknet und eingeengt.

Man erhält 17 g (62 % d. Th.) N-(2-Furancarbonyl)-N-(2'-methyl-4'-brom-1'-naphthyl)-alanin-methylester als farbloses Harz.

IR-Spektrum (Film) : 3 035, 2 930, 1 730, 1 640, 1 450, 1 360, 1 235, 1 190, 1 126, 1 092, 908, 810.

Beispiel 6

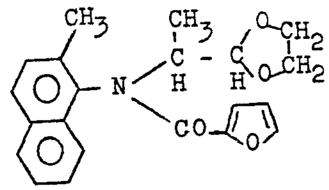

14,2 g (0,04 Mol) 2-[N-(2-Methyl-1-naphthyl)-N-(2-furancarbonyl)]-aminopropanal-dimethylacetal (Beispiel Nr. 23)

3 ml Ethylenglykol und 0,2 g p-Toluolsulfonsäure in 80 ml Toluol werden bei 80 °C 8 Stunden gerührt. Dabei wird das entstehende Methanol in einem schwachen Stickstoff-Strom ständig aus dem Reaktionsgemisch vertrieben. Nach Abkühlen und dreimaligen Waschen mit je 60 ml Wasser wird die organische Lösung mit Kohle entfärbt und im Vakuum eingeengt. Der Rückstand kristallisiert nach Zugabe von 10 ml kaltem Ether. Man erhält 9 g (64 % d. Th.) 2-Furancarbonsäure-N-[1-(1,3-dioxolan-2-yl)-2-methyl-ethyl]-N-2'-methyl-1'-naphthylamid als weiße Kristalle vom Schmelzpunkt 129-131 °C.

Beispiel 7

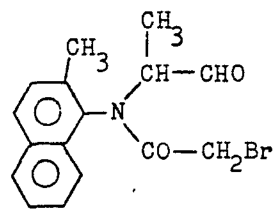

7,6 g (0,02 Mol) 2-[N-(2-Methyl-1-naphthyl)-N-bromacetyl]-aminopropanaldimethylacetal (Beispiel Nr. 12) gelöst in 50 ml Methylenchlorid werden 1 Stunde mit 100 ml 2N-Salzsäure bei Raumtemperatur kräftig gerührt. Die organische Phase wird abgetrennt, mit 100 ml Wasser gewaschen, getrocknet und eingeengt. Man erhält 5,3 g (80 % d. Th.) 2-[N-(2-Methyl-1-naphthyl)-N-bromacetyl]-aminopropionaldehyd als hellgelbes Öl.

IR (Film) : 3 038, 2 970, 2 820, 2 710, 1 720, 1 645, 1 490, 1 365, 1 203, 810, 778, 742, 640 $cm^{-1}$.

Analog werden folgende Verbindungen hergestellt :

Tabelle 1

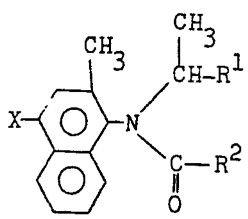

| Beispiel Nr. | X | $R^1$ | $R^2$ | Fp. °C | IR(Film) [$cm^{-1}$] |
|---|---|---|---|---|---|
| 8 | H | $-CH(OCH_3)_2$ | $-CH_3$ | Öl | 3042, 2980, 2930, 1645, 1448, 1360, 1295, 1148, 1120, 1062, 945, 814, 786, 747. |
| 9 | H | $-CH(OCH_3)_2$ | $-C_2H_5$ | Öl | 2972, 2928, 1642, 1445, 1365, 1256, 1120, 1080, 1060, 952, 811, 783, 744. |

Tabelle 1 (Fortsetzung)

| Beispiel Nr. | X | $R^1$ | $R^2$ | Fp. °C | IR(Film) [$cm^{-1}$] |
|---|---|---|---|---|---|
| 10 | H | $-COOCH_3$ | $-CH_2-Cl$ | öl | 3042, 2980, 2940, 1740, 1622, 1505, 1365, 1240, 1200, 1105, 1050, 815, 783, 748. |
| 11 | H | $-CH(OCH_3)_2$ | $-CH_2Cl$ | 80-82 | |
| 12 | H | $-COOCH_3$ | $-O-CH_3$ | Harz | 3040, 2940, 2910, 1732, 1690, 1430, 1325, 1190, 1066, 1025, 810, 785, 772, 742. |
| 13 | H | $-CH(OCH_3)_2$ | $-CH_2Br$ | Harz | 3018, 2912, 2810 1645, 1585, 1556, 1415, 1256, 1235, 1138, 1085, 810, 780, |
| 14 | H | $-COOCH_3$ | $-C_2H_5$ | öl | 3045, 2972, 1720, 1648, 1440, 1390, 1260, 1170, 1130, 1075, 812, 780, 644 |
| 18 | H | 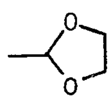 | $-CH_2-O-CH_3$ | 107-109 | |
| 23 | H | $-CH(OCH_3)_2$ | 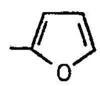 | öl | 2968, 2925, 1625, 1555, 1455, 1376, 1305, 1174, 1100, 1058, 810, 795, 779, 750. |
| 24 | H | $-COOCH_3$ | 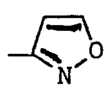 | Harz | 3108, 3038, 2935, 1726, 1635, 1532, 14.., 1330, 1200, 1100, 1042, 875, 856, 810, 796, 778, 758, 742. |
| 25 | H | $-CH(OCH_3)_2$ | 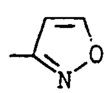 | Harz | 3105, 3040, 2930, 1640, 1544, 1438, 180., 1200, 1100, 1058, 835, 810, 795, 778, 75., 743. |

Tabelle 1 (Fortsetzung)

| Beispiel Nr. | X | $R^1$ | $R^2$ | Fp. °C | IR(Film) [$cm^{-1}$] |
|---|---|---|---|---|---|
| 26 | H | $-COOCH_3$ | 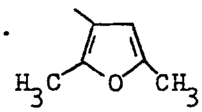 | Harz | 3038, 2970, 2935, 2908, 1725, 1615, 156., 1420, 1310, 1292, 1200, 1130, 1022, 810, 782, 763, 745. |
| 27 | H | $-COOCH_3$ | 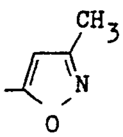 | Harz | 3030, 2932, 1728, 1640, 1560, 1430, 1135, 1290, 1210, 1188, 810, 779, 745. |

Die neuen Wirkstoffe zeigen eine starke fungitoxische Wirksamkeit gegen phytopathogene Pilze, insbesondere aus der Klasse der Phycomyceten. Die neuen Verbindungen sind daher beispielsweise geeignet zur Bekämpfung von Phytophthora infestans an Tomaten und Kartoffeln, Phytophthora parasitica an Erdbeeren, Phytophthora cactorum an Äpfeln, Pseudoperonospora cubensis an Gurken, Pseudoperonosppora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben, Erysiphe graminis an Getreide, Uncinula necator an Reben, Podophaera leucotricha an Äpfeln, Sphaerotheca fuliginea an Rosen, Erysiphe cichoriacearum an Gurken. Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 und 5 kg Wirkstoff je ha. Ein Teil der Wirkstoffe zeigt kurative Eigenschaften, d. h. die Anwendung der Mittel kann noch nach erfolgter Infektion der Pflanzen durch die Krankheitserreger vorgenommen werden, um einen sicheren Bekämpfungserfolg zu erzielen. Wirkstoffteilchen mit einer Teilchengröße kleiner als 5 µ zeigen eine gute fungizide Wirkung.

Darüber hinaus sind viele der neuen Verbindungen systemisch wirksam, so daß über die Wurzelbehandlung auch ein Schutz oberirdischer Pflanzenteile möglich ist.

Die erfindungsgemäßen Wirkstoffe können auch zusammen mit anderen Wirkstoffen, z. B. Herbiziden, Insektiziden, Wachstumsregulatoren und Fungiziden oder auch mit Düngemitteln vermischt und ausgebracht werden. In vielen Fällen erhält man bei der Mischung mit Fungiziden auch eine Vergrößerung des fungiziden Wirkungsspektrums ; bei einer Anzahl dieser Fungizidmischungen treten auch synergistische Effekte auf, d. h. die fungizide Wirksamkeit des Kombinationsproduktes ist größer als die der addierten Wirksamkeiten der Einzelkomponenten. Eine besonders günstige Vergrößerung des Wirkungsspektrums wird mit folgenden Fungiziden erzielt :

Manganethylenbisdithiocarbamat
Mangan-Zinkethylenbisdithiocarbamat
Ammoniak-Komplex von Zink-(N,N-ethylen-bis-dithiocarbamat)
N-Trichlormethylthio-tetrahydrophthalimid
N-Trichlormethyl-phthalimid
5-Äthoxy-3-trichlormethyl-1,2,3-thiadiazol
2-Methoxycarbonylamino-benzimidazol
2-Rhodanmethylthiobenzthiazol
1,4-Dichlor-2,5-dimethoxybenzol
2,3-Dichlor-6-methyl-1,4-oxathiin-5-carbonsäureanilid
2-Methyl-5,6-dihydro-4-H-pyran-3-carbonsäure-anilid
2,4,5-Trimethyl-furan-3-carbonsäureanilid
2-Methyl-furan-3-carbonsäureanilid
2,5-Dimethyl-furan-3-carbonsäurecyclohexylamid

N-Cyclohexyl-N-methoxy-2,5-dimethyl-furan-3-carbonsäure-amid
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Wirkstoffen kombiniert werden können, sind beispielsweise :

Dithiocarbamate und deren Derivate, wie
Ferridimethyldithiocarbamat
Zinkdimethyldithiocarbamat
Zinkethylenbisdithiocarbamat
Tetramethylthiuramdisulfide
Zink-(N,N-propylen-bis-dithiocarbamat)
Ammoniak-Komplex von Zink-(N,N'-propylen-bis-dithiocarbamat) und
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat

heterocyclische Strukturen, wie
2-Heptadecyl-2-imidazolin-acetat
2,4-Dichlor-6-(o-chloranilino)-s-triazin
0,0-Diethyl-phthalimidophonothioat
5-Amino-1-(bis-(dimethylamino)-phosphinyl)-3-phenyl-1,2,4-triazol)
2,3-Dicyano-1,4-dithiaanthrachinon
2-THio-1,3-dithio-(4,5-b)-chinoxalin
1-(Butylcarbamoyl)-2-benzimidazol-carbaminsäuremethylester
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4-dioxid
2,3-Dihydro-5-carboxanilido-6-methyl-1,4-oxathiin
2-(Furyl-(2))-benzimidazol
Piperazin-1,4-diylbis-(1-(2,2,2-trichlor-ethyl)-formamid
2-(Thiazolyl-(4)-benzimidazol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol sowie verschiedene Fungizide, wie
Dodecylguanidinacetat
3-(3-(3,5-Dimethyl-2-oxycyclohexyl)-2-hydroxyethyl)-glutarimid
Hexachlorbenzol
N-Dichlorfluormethylthio-N',N'-dimethyl-N-phenyl-schwefelsäurediamid
2,5-Dimethyl-furan-3-carbonsäureanilid
2-Methyl-benzoesäure-anilid
2-Jod-benzoesäure-anilid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
α-(2-Chlorphenyl)-α-(4-chlorphenyl)-5-pyrimidin-methanol.

Die neuen Wirkstoffe werden beispielsweise in Form von direkt versprühbaren Lösungen, Pulvern, Suspensionen, auch hochprozentige wäßrige, ölige oder sonstige Suspensionen oder Dispersionen, Emulsionen, Öldispersionen, Pasten, Stäubemitteln, Streumitteln, Granulaten, durch Versprühen, Vernebeln, Verstäuben, Verstreuen, Beizen oder Gießen angewendet. Die Aufwendungsformen richten sich ganz nach den Verwendungszwecken ; sie sollten in jedem Fall möglichst die feinste Verteilung der neuen Wirkstoffe gewährleisten. Die Teilchengröße kann die fungizide Wirkung des Wirkstoffs beeinflussen in dem Sinne, daß mit zunehmender Feinheit der Wirkstoffteilchen eine Verbesserung der fungiziden Wirksamkeit festgestellt wird.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner

Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate z. B. Methanol, Ethanol, Propanaol, Butanol, Chloroform, Tetrachlorkohlenstoff, Cyclohexanol, Cyclohexanon, Chlorbenzol, Isophoron usw., stark polare Lösungsmittel, wie z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen in Betracht: Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol-, Octylphenol-, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehle, Cellulosepulver und andere feste Trägerstoffe.

Für die folgenden Versuche wurden als bekannte Vergleichswirkstoffe die folgenden Verbindungen verwendet.

N-Trichlormethylthio-phthalimid (Verbindung A)
N-Trichlormethylthio-tetrahydrophthalimid (Verbindung B)
Zink-ethylen-1,2-bis-dithiocarbamat (Verbindung C).

## Versuch 1

Wirksamkeit gegen Auflaufkrankheiten an Erbsen

100-g-Proben Erbsensaatgut der Sorte « Senator » werden in Glasflaschen etwa 5 Minuten lang mit 300 mg (= 0,3 Gew.-%) Beizmittelaufbereitungen, die 40 % Wirkstoff in der Trockensubstanz enthalten, sorgfältig gebeizt. Danach werden jeweils 100 Samen in Saatkisten 3 cm tief und mit einem Abstand von 3 bis 5 cm in eine Komposterde eingesät, die eine starke natürliche Verseuchung mit den Pilzen Pythium spec., Aphanpmyces spec. und Fusarium oxysporum aufweist. Die Kästen werden im Gewächshaus bei Temperaturen von 17 bis 20 °C aufgestellt. Nach einer Versuchsdauer von 21 Tagen wird die Anzahl gesunder Erbsenpflanzen ermittelt.

| Wirkstoff (Beizmittelaufbereitung) | gesunde Pflanzen nach 21 Tagen in Komposterde % |
|---|---|
| 2 | 86 |
| 10 | 84 |
| Verbindung B (Vergleichsmittel) | 70 |
| Kontrolle (unbehandelt) infizierte Komposterde | 18 |
| Kontrolle (unbehandelt) sterilisierte Komposterde | 92 |

Versuch 2

Wirksamkeit gegen Erysiphe graminis an Weizen

Blätter von in Töpfen gewachsenen Weizenkeimlingen der Sorte « Jubilar » werden mit wäßrigen Suspensionen aus 80 % (Gewichtsprozent) Wirkstoff und 20 % Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22 °C und 75 bis 80 % relativer Luftfeuchtigkeit aufgestellt. Nach 10 Tagen wird das Ausmaß der Mehltaupilzentwicklung ermittelt.

| Wirkstoff | Befall der Blätter nach Spritzungen mit x%iger Wirkstoffbrühe | | |
|---|---|---|---|
| | x = 0,025 | 0,012 | 0,006 |
| 5 | 0 | 2 | 4 |
| 8 | 1 | 1 | 3-4 |
| 12 | 0 | 0 | 0 |
| 26 | 1 | 1 | 1 |
| Kontrolle (unbehandelt) | 5 | | |

0 = kein Pilzbefall, abgestuft bis 5 = Totalbefall

Versuch 3

Wirksamkeit gegen Phytophthora infestans an Tomaten

Blätter von Topfpflanzen der Sorte « Große Fleischtomate » werden mit wäßrigen Suspensionen, die 0,025 % (Gewichtsprozent) Wirkstoff enthalten, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18 °C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

| Wirkstoff | Befall der Blätter nach Spritzung mit 0,025%iger Wirkstoffbrühe |
|---|---|
| 4 | 0 |
| 8 | 0 |
| 24 | 1 |
| Verbindung C (Vergleichsmittel) | 2-3 |
| Kontrolle (unbehandelt) | 5 |

0 = kein Pilzbefall, abgestuft bis 5 = Totalbefall

Versuch 4

Wirksamkeit gegen Plasmopara viticola an Reben

Blätter von Topfreben der Sorte « Müller-Thurgau » werden mit wäßrigen Suspensionen, die 0,025 % (Gewichtsprozent) Wirkstoff enthalten, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach kommen die Reben zunächst für 16 Stunden in eine wasserdampfgesättigte Kammer bei 24 °C und anschließend 8 Tage in ein Gewächshaus mit Temperaturen zwischen 20 und 30 °C. Nach dieser Zeit werden die Pflanzen zur Beschleunigung des Sporangienträgerausbruches abermals während 16 Stunden in der feuchten Kammer aufgestellt. Dann erfolgt die Beurteilung des Ausmaßes des Pilzsausbruches auf den Blattunterseiten.

| Wirkstoff | Befall der Blätter nach Spritzung mit 0,025%iger Wirkstoffbrühe |
|---|---|
| 10 | 0 |
| Verbindung A (Vergleichsmittel) | 2 |
| Kontrolle (unbehandelt) | 5 |

0 = kein Pilzbefall, abgestuft bis 5 = Totalbefall

Beispiele für Zubereitungen sind :

I. Man vermischt 90 Gew.-Teile der Verbindung 1 mit 10 Gew.-Teilen N-Methyl-alpha-pyrrolidon und erhält eine Lösung, die zur Anwendung in Form kleinster Tropfen geeignet ist.

II. 10 Gew.-Teile der Verbindung 2 werden in einer Mischung gelöst, die aus 90 Gew.-Teilen Xylol, 6 Gewichtsteilen des Anlagerungsproduktes von 8 bis 10 Mol Ethylenoxid an 1 Mol Ölsäure-N-monoethanolamin, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

III. 20 Gew.-Teile der Verbindung 3 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.

IV. 20 Gew.-Teile der Verbindung 1 werden in einer Mischung gelöst, die aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.

V. 80 Gew.-Teile der Verbindung 2 werden mit 3 Gew.-Teilen des Natriumsalzes der Diisobutylnaphthalin-alphasulfonsäure, 10 Gew.-Teilen des Natriumsalzes einer Ligninsulfonsäure aus einer Sulfitablauge und 7 Gew.-Teilen pulverförmigen Kieselsäuregel gut vermischt und in einer Hammermühle vermahlen. Durch feines Verteilen der Mischung in Wasser erhält man eine Spritzbrühe.

VI. 3 Gew.-Teile der Verbindung 3 werden mit 97 Gew.-Teilen feinteiligem Kaolin innig vermischt. Man erhält auf diese Weise ein Stäubemittel, das 3 Gew.-% des Wirkstoffs enthält.

VII. 30 Gew.-Teile der Verbindung 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung 2 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit 100 000 Gew.-Teilen Wasser erhält man eine wäßrige Dispersion, die 0,04 Gew.-% Wirkstoff enthält.

IX. 20 Teile der Verbindung 3 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

**Ansprüche** (für die Vertragsstaaten : BE, CH, DE, FR, GB, IT, LI, LU, NL, SE)

1. N-substituiertes 2-Methylnaphthylamid der allgemeinen Formel

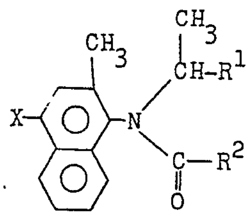

worin

$R^1$ eine

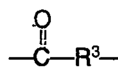—Gruppe

bedeutet, wobei $R^3$ Wasserstoff oder $C_1$-$C_4$-Alkoxy bedeutet und weiterhin $R^1$ eine

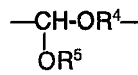—Gruppe

bedeutet, wobei $R^4$ und $R^5$ unabhängig voneinander einen $C_1$-$C_4$-Alkylrest oder $R^4$ und $R^5$ zusammen eine Alkylengruppe bedeuten, die zusammen mit dem Rest, dessen Substituenten sie sind, einen gegebenenfalls durch $C_1$-$C_4$-Alkyl oder Aryl substituierten 5- oder 6-gliedrigen zwei Sauerstoffatome enthaltenden heterocyclischen Ring bildet und $R^2$ einen gegebenenfalls durch Fluor, Chlor, Brom, Jod, $C_1$-$C_4$-Alkoxy, $C_1$-$C_4$-Alkylthio, 1-Imidazolyl, 1-Pyrazolyl, 1,2,4-Triazolyl-(1) oder Oxo (= O)-substituierten $C_1$-$C_4$-Alkyl-, ferner einen $C_2$-$C_5$-Alkenyl-, $C_2$-$C_5$-Alkinyl, $C_3$-$C_7$-Cycloalkyl-, $C_4$-$C_7$-Cycloalkenyl-, $C_1$-$C_5$-Alkoxyrest, einen Phenyl- oder gegebenenfalls durch Methyl substituierten Heteroarylrest bedeutet und X Wasserstoff, Methyl, Chlor oder Brom bedeutet, mit der Maßgabe, daß nicht gleichzeitig $R^3$ $C_1$-$C_4$-Alkoxy, X Wasserstoff und $R^2$ einen 2-Furylrest, einen $C_1$-$C_4$-Alkoxy-$C_1$-$C_2$-alkylrest, einen $C_1$-$C_4$-Alkylthio-$C_1$-$C_2$-alkylrest, einen $-CH_2$-1,2-pyrazolylrest oder einen $-CH_2$-1,2,4-Triazolylrest bedeuten.

2. Fungizid, enthaltend ein N-substituiertes 2-Methyl-naphthylamid gemäß Anspruch 1.

3. Verfahren zur Bekämpfung von pflanzenschädigenden Pilzen, dadurch gekennzeichnet, daß man die Pilze oder die vor Pilzbefall zu schützenden Pflanzen behandelt mit einem N-substituierten 2-Methylnaphthylamid gemäß Anspruch 1.

4. Verfahren zur Herstellung eines N-substituierten 2-Methylnaphthylamids gemäß Anspruch 1, dadurch gekennzeichnet, daß man ein 2-Methylnaphthylamin der Formel

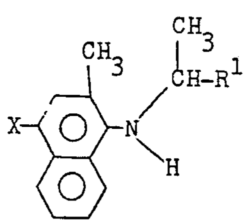

worin

$R^1$ und X die in Anspruch 1 angegebenen Bedeutungen haben, mit einem Säurederivat der Formel

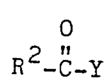

worin

Y Chlor, Brom oder den Rest

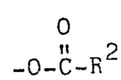

bedeutet und $R^2$ die in Anspruch 1 angegebene Bedeutung hat, gegebenenfalls in Gegenwart eines Lösungsmittels, gegebenenfalls unter Zusatz einer anorganischen oder organischen Base und gegebenenfalls unter Zusatz eines Reaktionsbeschleunigers bei Temperaturen zwischen − 10 und 100 °C umsetzt.

5. N-(3-Isoxazolyl-carbonyl)-N-(2'-methyl-1'-naphthyl)-alanin-methylester.

6. Fungizid, enthaltend den N-(3-Isoxazolyl-carbonyl)-N-(2'-methyl-1'-naphthyl)-alanin-methylester.

7. N-(5-Isoxazolyl-carbonyl)-N-(2'-methyl-1'-naphthyl)-alanin-methylester.

8. Fungizid, enthaltend den N-(5-Isoxazolyl-carbonyl)-N-(2'-methyl-1'-naphthyl)-alanin-methylester.

**Ansprüche** (für den Vertragsstaat AT)

1. Fungizid, enthaltend ein N-substituiertes 2-Methyl-naphthylamid der allgemeinen Formel

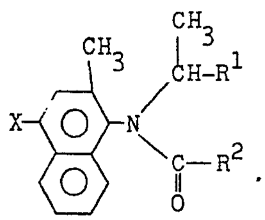

worin

$R^1$ eine

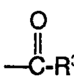—Gruppe

bedeutet, wobei $R^3$ Wasserstoff oder $C_1$-$C_4$-Alkoxy bedeutet und weiterhin $R^1$ eine

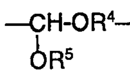—Gruppe

bedeutet, wobei $R^4$ und $R^5$ unabhängig voneinander einen $C_1$-$C_4$-Alkylrest oder $R^4$ und $R^5$ zusammen eine Alkylengruppe bedeuten, die zusammen mit dem Rest, dessen Substituenten sie sind, einen gegebenenfalls durch $C_1$-$C_4$ Alkyl oder Aryl substituierten 5- oder 6-gliedrigen zwei Sauerstoffatome enthaltenden heterocyclischen Ring bildet und $R^2$ einen gegebenenfalls durch Fluor, Chlor, Brom, Jod, $C_1$-$C_4$-Alkoxy, $C_1$-$C_4$-Alkylthio, 1-Imidazolyl, 1-Pyrazolyl, 1,2,4-Triazolyl-(1) oder oxo (= O)-substituierten $C_1$-$C_4$-Alkyl-, ferner einen $C_2$-$C_5$-Alkenyl-, $C_2$-$C_5$-Alkinyl, $C_3$-$C_7$-Cycloalkyl, $C_4$-$C_7$-Cycloalkenyl, $C_1$-$C_5$-Alkoxyrest, einen Phenyl- oder gegebenenfalls durch Methyl substituierten Heteroarylrest bedeutet und X Wasserstoff, Methyl, Chlor oder Brom bedeutet, mit der Maßgabe, daß nicht gleichzeitig $R^3$ $C_1$-$C_4$-Alkoxy, X Wasserstoff und $R^2$ einen 2-Furylrest, einen $C_1$-$C_4$-Alkoxy-$C_1$-$C_2$-alkylrest, einen $C_1$-$C_4$-Alkylthio-$C_1$-$C_2$-alkylrest, einen $-CH_2$-1,2-pyrazolylrest oder einen $-CH_2$-1,2,4-Triazolylrest bedeuten.

2. Fungizid, enthaltend einen festen oder flüssigen Trägerstoff und ein N-substituiertes 2-Methylnaphthylamid wie im Anspruch 1 definiert.

3. Verfahren zur Bekämpfung von pflanzenschädigenden Pilzen, dadurch gekennzeichnet, daß man die Pilze oder die vor Pilzbefall zu schützenden Pflanzen behandelt mit einem N-substituierten 2-Methylnaphthylamid wie im Anspruch 1 definiert.

4. Verfahren zur Herstellung eines N-substituierten 2-Methylnaphthylamids wie im Anspruch 1 definiert, dadurch gekennzeichnet, daß man ein 2-Methylnaphthylamin der Formel

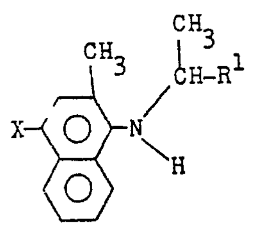

worin

$R^1$ und X die in Anspruch 1 angegebenen Bedeutungen haben, mit einem Säurederivat der Formel

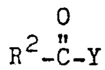

worin

Y Chlor, Brom oder den Rest

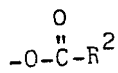

bedeutet und $R^2$ die in Anspruch 1 angegebene Bedeutung hat, gegebenenfalls in Gegenwart eines Lösungsmittels, gegebenenfalls unter Zusatz einer anorganischen oder organischen Base und gegebenenfalls unter Zusatz eines Reaktionsbeschleunigers bei Temperaturen zwischen − 10 und 100 °C umsetzt.

5. Fungizid, enthaltend den N-(3-Isoxazolyl-carbonyl)-N-(2′-methyl-1′-naphthyl)-alanin-methylester.

6. Fungizid, enthaltend den N-(5-Isoxazolyl-carbonyl)-N-(2′-methyl-1′-naphthyl)-alanin-methylester.

**Claims** (for the Contracting States : BE, CH, DE, FR, GB, IT, LI, LU, NL, SE)

1. An N-substituted 2-methylnaphthylamide of the general formula

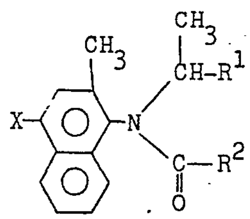

where

$R_1$ is

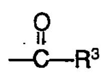

$R^3$ being hydrogen or $C_1$-$C_4$-alkoxy, or $R^1$ is

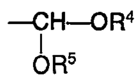

$R^4$ and $R^5$ independently of one another being $C_1$-$C_4$-alkyl or $R^4$ and $R^5$ together being an alkylene group which, together with the radical on which they are present as substituents, forms a 5-membered or 6-membered heterocyclic ring which contains two oxygen atoms and is unsubstituted or substituted by $C_1$-$C_4$-alkyl or aryl, and $R^2$ is $C_1$-$C_4$-alkyl which is unsubstituted or substituted by fluorine, chlorine, bromine, iodine, $C_1C_4$-alkoxy, $C_1$-$C_4$-alkylthio, imidazol-1-yl- pyrazol-1-yl, 1,2,4-triazol-1-yl or oxo (= O), or is $C_2$-$C_5$-alkenyl, $C_2$-$C_5$-alkynyl, $C_3$-$C_7$-cycloalkyl, $C_4$-$C_7$-cycloalkenyl, $C_1$-$C_5$-alkoxy, phenyl or unsubstituted or substituted hetaryl, and X is hydrogen, methyl, chlorine or bromine, with the proviso that the meanings $R^3$ = $C_1$-$C_4$-alkoxy, X = hydrogen, and $R^2$ = fur-2-yl, $C_1$-$C_4$-alkoxy-$C_1$-$C_2$-alkyl, $C_1$-$C_4$-alkylthio-$C_1$-$C_2$-alkyl, -$CH_2$-1,2-pyrazole or -$CH_2$-1,2,4-triazolyl do not occur simultaneously.

2. A fungicide containing an N-substituted 2-methylnaphthylamide as claimed in claim 1.

3. A process for combatting plant-damaging fungi, wherein the fungi or the plants to be protected against fungus attack are treated with an N-substituted 2-methylnaphthylamide as claimed in claim 1.

4. A process for producing an N-substituted 2-methylnaphthylamide as claimed in claim 1, wherein a 2-methylnaphthylamine of the formula

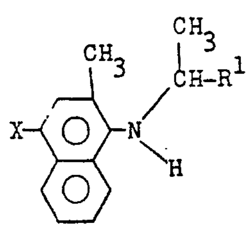

where

$R^1$ and X have the meanings given in claim 1, is reacted with an acid derivative of the formula

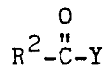

where

Y denotes chlorine bromine or

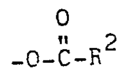

$R^2$ having the meanings given in claim 1, in the presence or absence of a solvent, with or without addition of an inorganic or organic base and with or without addition of a reaction accelerator, at from − 10 to 100 °C.

5. Methyl N-(3-isoxazolyl-carbonyl)-N-(2′-methyl-1′-naphthyl)-alanate.

6. A fungicide containing methyl N-(3-isoxazolyl-carbonyl)-N-(2′-methyl-1′-naphthyl)-alanate.

7. Methyl N-(5-isoxazolyl-carbonyl)-N-(2′-methyl-1′-naphthyl)-alanate.

8. A fungicide containing methyl N-(5-isoxazolyl-carbonyl)-N-(2′-methyl-1′-naphthyl)-alanate.

**Claims** (for the Contracting State AT)

1. A fungicide containing an N-substituted 2-methylnaphthylamide of the general formula

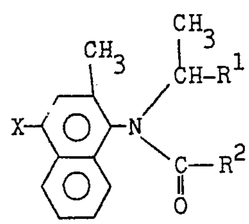

where

$R_1$ is

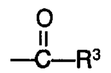

$R^3$ being hydrogen or $C_1$-$C_4$-alkoxy, or $R^1$ is

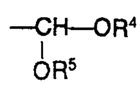

$R^4$ and $R^5$ independently of one another being $C_1$-$C_4$-alkyl or $R^4$ and $R^5$ together being an alkylene group which, together with the radical on which they are present as substituents, forms a 5-membered or 6-membered heterocyclic ring which contains two oxygen atoms and is unsubstituted or substituted by $C_1$-$C_4$-alkyl or aryl, and $R^2$ is $C_1$-$C_4$-alkyl which is unsubstituted or substituted by fluorine, chlorine, bromine, iodine, $C_1$-$C_4$-alkoxy, $C_1$-$C_4$-alkylthio, imidazol-1,yl- pyrazol-1-yl, 1,2,4-triazol-1-yl or oxo (= 0), or is $C_2$-$C_5$-alkenyl, $C_2$-$C_5$-alkynyl, $C_3$-$C_7$-cycloalkyl, $C_4$-$C_7$-cycloalkenyl, $C_1$-$C_5$-alkoxy, phenyl or unsubstituted or substituted hetaryl, and X is hydrogen, methyl, chlorine or bromine, with the proviso that the meanings $R^3$ = $C_1$-$C_4$-alkoxy, X = hydrogen, and $R^2$ = fur-2-yl, $C_1$-$C_4$-alkoxy-$C_1$-$C_2$-alkyl, $C_1$-$C_4$-alkylthio-$C_1$-$C_2$-alkyl, $-CH_2$-1,2-pyrazole or $-CH_2$-1,2,4-triazolyl do not occur simultaneously.

2. A fungicide containing a solid or liquid carrier and an N-substituted 2-methylnaphthylamide as defined in claim 1.

3. A process for combatting plant-damaging fungi, wherein the fungi or the plants to be protected against fungus attack are treated with an N-substituted 2-methylnaphthylamide as defined in claim 1.

4. A process for producing an N-substituted 2-methylnaphthylamide as defined in claim 1, wherein a 2-methylnaphthylamine of the formula

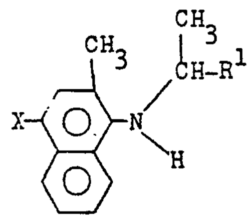

where
R¹ and X have the meanings given in claim 1, is reacted with an acid derivative of the formula

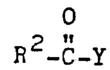

where
Y denotes chlorine, bromine or

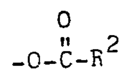

$R^2$ having the meanings given in claim 1, in the presence or absence of a solvent, with or without addition of an inorganic or organic base and with or without addition of a reaction accelerator, at from − 10 to 100 °C.

5. A fungicide containing methyl N-(3-isoxazolyl-carbonyl)-N-(2'methyl-1'-naphthyl)-alanate.

6. A fungicide containing methyl N-(5-isoxazolyl-carbonyl)-N-(2'-methyl-1'-naphthyl)-alanate.

**Revendications** (pour les Etats contractants : BE, CH, DE, FR, GB, IT, LI, LU, NL, SE)

1. Méthyl-2 naphtyl-amide N-substitué de la formule générale

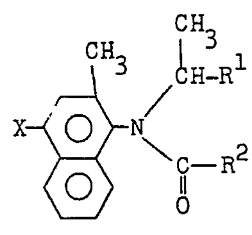

dans laquelle
$R^1$ représente un groupe

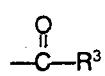

où $R^3$ désigne un atome d'hydrogène ou un radical alcoxy en $C_1$ à $C_4$ ou un groupe

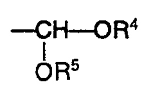

où $R^4$ et $R^5$ désignent, indépendamment l'un de l'autre, un radical alcoyle en $C_1$ à $C_4$, ou $R^4$ et $R^5$ forment ensemble un groupe alcoylène qui forme avec le reste, dont ils sont les substituants, un noyau hétérocyclique penta- ou hexagonal, contenant deux atomes d'oxygène, éventuellement substitué par des groupes alcoyle en $C_1$ à $C_4$ ou aryle ; $R^2$ représente un radical alcoyle en $C_1$ à $C_4$ éventuellement substitué par le fluor, le chlore, le brome ou l'iode ou des groupes alcoxy en $C_1$ à $C_4$, alcoyl-thio en $C_1$ à $C_4$, 1-imidazolyle, 1-pyrazolyle, 1,2,4-triazolyle-(1) ou oxo (= O), ou encore un groupe alcényle en $C_2$ à $C_5$, alcynyle en $C_2$ à $C_5$, cycloalcoyle en $C_3$ à $C_7$, cycloalcényle en $C_4$ à $C_7$, alcoxy en $C_1$ à $C_4$ ou phényle ou un reste hétéro-aryle éventuellement méthyl-substitué et X représente un atome d'hydrogène, de chlore ou de brome ou un radical méthyle, avec la condition que l'on n'ait pas simultanément les significations

$R^3$ = alcoxy en $C_1$ à $C_4$, X = H et $R^2$ = 2-furyle, alcoxy (en $C_1$ à $C_4$)-alcoyle (en $C_1$ ou $C_2$), alcoyl-thio (en $C_1$ à $C_4$)-alcoyle (en $C_1$ ou $C_2$), $-CH_2$-1,2-pyrazolyle ou $-CH_2$-1,2,4-triazolyle.

2. Fongicide, contenant un méthyl-2 naphtyl-amide N-substitué selon la revendication 1.

3. Procédé de lutte contre les champignons phytopathologiques, caractérisé en ce que l'on traite les champignons ou les plantes à protéger des champignons avec un méthyl-2 naphtyl-amide N-substitué selon la revendication 1.

4. Procédé de préparation d'un méthyl-2 naphtyl-amide N-substitué selon la revendication 1, caractérisé en ce que l'on fait réagir une méthyl-2 naphtyl-amine de la formule

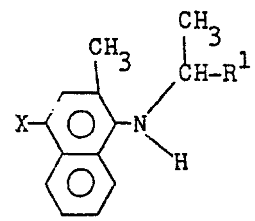

dans laquelle

$R^1$ et X possèdent les significations définies dans la revendication 1, à des températures entre − 10 et 100 °C avec un dérivé d'acide de la formule

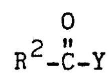

dans laquelle

Y représente un atome de chlore ou de brome ou un groupe

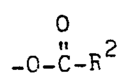

et $R^2$ possède la signification définie dans la revendication 1, cette réaction pouvant, le cas échéant, être effectuée en présence d'un solvant et (ou) d'une base inorganique ou organique et (ou) d'un accélérateur de la réaction.

5. Le N-(isoxazolyl-3 carbonyl) N-(méthyl-2′ naphtyl-1′) alanate de méthyle.

6. Fongicide, contenant du N-(isoxazolyl-3-carbonyl) N-(méthyl-2′) alanate de méthyle.

7. Le N-(isoxazolyl-5 carbonyl) N-(méthyl-2′ naphtyl-1′) alanate de méthyle.

8. Fongicide, contenant du N-(isoxazolyl-3 carbonyl) N-(méthyl-2′ naphtyl-1′) alanate de méthyle.

**Revendications** (pour l'Etat contractant : AT)

1. Fongicide contenant un méthyl-2 naphtyl-amide N-substitué de la formule générale

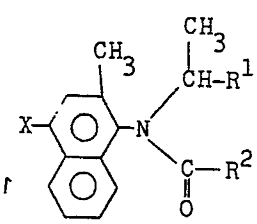

dans laquelle

$R^1$ représente un groupe

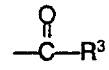

où $R^3$ désigne un atome d'hydrogène ou un radical alcoxy en $C_1$ à $C_4$ ou un groupe

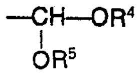

où $R^4$ et $R^5$ désignent, indépendamment l'un de l'autre, un radical alcoyle en $C_1$ à $C_4$, ou $R^4$ et $R^5$ forment ensemble un groupe alcoylène qui forme avec le reste, dont ils sont les substituants, un noyau hétérocyclique penta- ou hexagonal, contenant deux atomes d'oxygène, éventuellement substitué par des groupes alcoyle en $C_1$ à $C_4$ ou aryle ; $R^2$ représente un radical alcoyle en $C_1$ à $C_4$ éventuellement substitué par le fluor, le chlore, le brome ou l'iode ou des groupes alcoxy en $C_1$ à $C_4$, alcoyl-thio en $C_1$ à $C_4$, 1-imidazolyle, 1-pyrazolyle, 1,2,4-triazolyle-(1) ou oxo (= O), ou encore un groupe alcényle en $C_2$ à $C_5$, alcynyle en $C_2$ à $C_5$, cycloalcoyle en $C_3$ à $C_7$, cycloalcényle en $C_4$ à $C_7$, alcoxy en $C_1$ à $C_4$ ou phényle ou un reste hétéro-aryle éventuellement méthyl-substitué et X représente un atome d'hydrogène, de chlore ou de brome ou un radical méthyle, avec la condition que l'on n'ait pas simultanément les significations $R^3$ = alcoxy en $C_1$ à $C_4$, X = H et $R^2$ = 2-furyle, alcoxy (en $C_1$ à $C_4$)-alcoyle (en $C_1$ ou $C_2$), alcoyl-thio (en $C_1$ à $C_4$)-alcoyle (en $C_1$ ou $C_2$), $-CH_2$-1,2-pyrazolyle ou $-CH_2$-1,2,4-triazolyle.

2. Fongicide, contenant un excipient solide ou un véhicule liquide et un méthyl-2 naphtyl-amide N-substitué tel que défini dans la revendication 1.

3. Procédé de lutte contre les champignons phytopathologiques, caractérisé en ce que l'on traite les champignons ou les plantes à protéger des champignons avec un méthyl-2 naphtyl-amide N-substitué tel que défini dans la revendication 1.

4. Procédé de préparation d'un méthyl-2 naphtyl-amide N-substitué selon la revendication 1, caractérisé en ce que l'on fait réagir une méthyl-2 naphtyl-amine de la formule

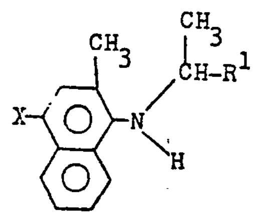

dans laquelle

$R^1$ et X possèdent les significations définies dans la revendication 1, à des températures entre − 10 et 100 °C avec un dérivé d'acide de la formule

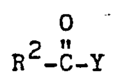

dans laquelle

Y représente un atome de chlore ou de brome ou un groupe

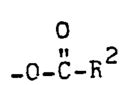

et $R^2$ possède la signification définie dans la revendication 1, cette réaction pouvant, le cas échéant, être effectuée en présence d'un solvant et (ou) d'une base inorganique ou organique et (ou) d'un accélérateur de la réaction.

5. Fongicide, contenant du N-(isoxazolyl-3 carbonyl) N-(méthyl-2' naphtyl-1') alanate de méthyle.

6. Fongicide, contenant du N-(isoxazolyl-5 carbonyl) N-(méthyl-2' naphtyl-1') alanate de méthyle.